# EUROPEAN PATENT SPECIFICATION

(11) **EP 4 308 678 B1**
(45) Date of publication and mention of the grant of the patent: **06.05.2026**
(21) Application number: 22714707.1
(22) Date of filing: 16.03.2022
(51) Int. Cl.: C12M 1/36, C12M 1/00, G01N 21/01, G01N 21/85, G01N 21/03, C12M 3/06

(54) **ROCKING BIOREACTOR WITH INTEGRATED MONITORING PROBE SYSTEM**
SCHWINGENDER BIOREAKTOR MIT INTEGRIERTEM ÜBERWACHUNGSSONDENSYSTEM
BIORÉACTEUR À BASCULE AVEC SYSTÈME DE SONDE DE SURVEILLANCE INTÉGRÉ

(30) Priority: 17.03.2021 US 202163162304 P
(43) Date of publication of application: 24.01.2024
(73) Proprietor: Nirrin Technologies, Inc., Billerica MA 01821 (US)
(72) Inventor: HASSELL, Bryan A., Cambridge, Massachusetts 02140 (US); MARCHESSAULT, David, P., Hopkinton, Massachusetts 01748 (US)
(74) Representative: HGF
(86) International application number: PCT/US2022/020605
(87) International publication number: WO 2022/197841

(56) References cited:
- EP-A1- 3 045 521
- US-A1- 2005 264 817
- US-A1- 2012 291 238
- US-A1- 2021 371 785

## Description

### RELATED APPLICATIONS

This application claims the benefit under 35 USC 119(e) of U.S. Provisional Application No. 63/162,304, filed on March 17, 2021.

### BACKGROUND OF THE INVENTION

Many processes in the chemical, biochemical, pharmaceutical, food, beverage and in other industries require some type of monitoring.

Sensors have been developed and are available to measure pH, dissolved oxygen (DO), temperature or pressure in-situ and in real-time. Common techniques for detecting chemical constituents include high performance liquid chromatography (HPLC), gas chromatography-mass spectroscopy (GCMS), or enzyme- and reagent-based electrochemical methods.

While considered accurate, many existing approaches are conducted off-line, tend to be destructive with respect to the sample, often require expensive consumables and/or take a long time to complete. In many cases, the equipment needed to perform these analyses is expensive, involves complex calibrations, and trained operators. Procedures may be time- and labor-intensive, often mitigated by decreasing the sampling frequency of a given process, thus reducing the data points. Often, samples are run in batches, after the process has been completed, yielding little or no feedback for adjusting conditions on an ongoing basis. Drawbacks such as these can persist even with automated sampling operations.

Various optical spectroscopy approaches are available to assess components, also referred to as analytes, in a sample. Among these, probably the most common is absorption spectroscopy. Incident light excites electrons of the analyte from a low energy ground state into a high energy, excited state, and the energy can be absorbed by both non-bonding n-electrons and π-electrons within a molecular orbital. Absorption spectroscopy can be performed in the ultraviolet, visible, and/or infrared region, with analytes of varying material phases and composition being interrogated by specific wavelengths or wavelength bands of light. The resulting transmitted light is then used to resolve the absorbed spectra, to determine the analyte's or sample's composition, temperature, pH and/or other intrinsic properties for applications ranging from medical diagnostics, pharmaceutical development, food and beverage quality control, to list a few.

To this end, Hassell, et al. in U.S. Pat. Pub. No.: US 2021/0088433 describe an in-situ probe that can be inserted and/or maintained in a bioreactor and incorporates elements for interrogating as well as elements needed to analyze the contents of a bioreactor, e.g., in the NIR region of the electromagnetic spectrum. The analysis can be conducted in real time, in a nondestructive manner. EP 3 045 521 A1 discloses a system for monitoring a process within a bioreactor bag wherein a portion of the bag with its content is traversed by the light path of a turbidity sensor positioned outside the bag.

Another option is Raman spectroscopy, which works by the detection of inelastic scattering of typically monochromatic light from a laser.

### SUMMARY OF THE INVENTION

Robust, hands-free, non-destructive techniques for identifying and/or quantifying constituents in a given process in real time are highly desirable. Typically, the process is conducted in a vessel, e.g., a bioreactor. The contents of the bioreactor can change as the process unfolds and data collected at various stages can be used to monitor, adjust and/or control process parameters.

Whereas many existing approaches rely on removing and/or circulating cells in loops external to the process vessel, typically through a pumping system, an in-situ probe can reduce, minimize and often eliminate the exposure of the bag contents to external conditions. In addition, cells are prevented from being drawn into a pumping system, where they could become damaged. The low sheer rocking motion often used with flexible reactors, as well as the absence of stirrers, impellers and the like, are other factors that contribute to protecting cells from damage, while also minimizing contamination.

Nevertheless, a need continues to exist for methods and equipment designed to monitor processes conducted in modern bioreactors, and, in particular, flexible bioreactors, also referred to herein as "bag bioreactors" and/or "rocking bioreactors".

Advantageously, rocking reactors come in a wide range of sizes, to accommodate many applications and needs. Typically, pre-sterilized and often designed for single use, such reactors reduce the need for time-consuming vessel clean-up steps and simplify the process.

Embodiments described herein reduce manual intervention, increasing reproducibility from one run to the next, streamlining the process, and reducing the potential for errors.

Generally, bags for reactors, such as rocking reactors, are single use, flexible bags made of polymeric materials. They can be configured in a variety of sizes and are often pre-sterilized. Easy to handle, often inexpensive, these bags offer time and labor advantages. Nevertheless, they can also present some challenges, when wishing to analyze process ingredients, for example.

Described herein are approaches that employ a probe for monitoring a process conducted in a flexible bag of a rocking bioreactor. Specific examples include a bag insert or patch that is often disposable with the bag and a reusable probe module that is compatible with the insert. This module includes one or more source elements, one or more detector elements, and often a bench.

The bag patch can be fused, glued, adhered, welded or thermo-formed to the bag material, specifically a wall, such as a bottom wall of the bag.

The probe module is a detachable part that can be mated to the bag patch and then disassembled, e.g., for reuse.

With the probe in place, various contents of the rocking reactor can be analyzed spectroscopically, using, for instance near infrared absorption spectrometry. In many cases, the analysis process according to embodiments disclosed herein is simplified and/or accelerated.

Moreover, users can receive the bag and patch already assembled and even pre-gamma sterilized, allowing the users to simply insert the probe module when ready to collect data, with the probe module never touching the sample (and thus being re-usable) and without risk of compromising the sterile field of the bag.

In general, according to one aspect, the invention is directed to a system for monitoring a process conducted within a bag for a bioreactor, as defined in appended claim 1.

In embodiments, the bag is a flexible bag of a rocking bioreactor. The patch comprises a source hood and a detector hood. At least one of these hoods projects into the bag, defining the sample gap between the two hoods, which are each accessible from outside the bag. The probe module is then inserted into the patch such that the source hood receives a source assembly, housing the source element, and the detector hood receives a detector assembly, housing the detector element. Thus, the source assembly and the detector assembly are both secured to or integral with a common assembly base of the probe module. Sizes and shapes of the source assembly and the detector assembly and spacing between the source assembly and the detector assembly correspond to sizes and shapes of the source hood and the detector hood and the spacing between the hoods. The patch can be secured to the wall of the bag via welding, thermo-forming, or adhesive, for example. In one example, the bag and the patch are disposable or single-use components, and the probe module is reusable with different patches and bags. The patch can also comprise one or more window plates for facilitating the transmission of the light across the sample gap and between the source hood and detector hood.

In general, according to another aspect, the invention is directed to a method for monitoring a process within a bag as defined in appended claim 11.

In general, according to another aspect, the invention is directed to a bag for a bioreactor, as defined in appended claim 21.

In general, according to a further aspect, the invention is directed to a rocking bioreactor system, as defined in appended claim 22.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration. The principles and features of this invention may be employed in various and numerous embodiments within the scope of the invention which is defined by the appendend claims.

### BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:
FIGS. 1A and 1B are schematic side views illustrating the components and principles of operation of a rocking bioreactor;
FIG. 2 is a schematic side view of a rocking bioreactor including a bag disposed onto a rocking platform and fitted with an in-situ probe according to the present invention;
FIG. 3A is a schematic side cross sectional view of an exemplary bag to which the present invention is applicable;
FIG. 3B is a schematic side cross sectional view of the bag illustrated in FIG. 3A, showing a hole in a wall of the bag;
FIG. 4 is a schematic side cross sectional view of the in-situ probe illustrated in FIG. 2 according to one embodiment of the present invention, showing the probe disassembled from the bag;
FIG. 5 is a schematic side cross sectional view of the in-situ probe illustrated in FIG. 4, showing the probe in a fully assembled form;
FIG. 6 is a schematic side cross sectional view of the in-situ probe illustrated in FIG. 2 according to one embodiment of the present invention;
FIGS. 7A, 7B, and 7C are perspective view of an exemplary in-situ probe, specifically, the bag patch, the probe module, and the probe module inserted into the patch, respectively;
FIG. 8 is a cross sectional view of the in-situ probe illustrated in FIG. 2 according to an embodiment of the present invention;
FIG. 9 is a flow diagram illustrating the process by which the in-situ probe is used to monitor a process within a bag;
FIG. 10 is a schematic plan view of the in-situ probe showing how the patch can be advantageously positioned based on the direction of fluid motion in a rocking bioreactor;
FIG. 11 is a schematic side cross sectional view of the in-situ probe illustrated in FIG. 2 according to another embodiment of the present invention;
FIG. 12 is a schematic side cross sectional view of the in-situ probe illustrated in FIG. 2 according to another embodiment of the present invention;
FIG. 13 is a schematic side cross sectional view of the in-situ probe illustrated in FIG. 2 according to another embodiment of the present invention;
FIG. 14 is a schematic side cross sectional view of the in-situ probe illustrated in FIG. 2 according to another embodiment of the present invention;
FIG. 15 is a schematic side cross sectional view of the in-situ probe illustrated in FIG. 2 according to another embodiment of the present invention; and
FIG. 16 is a schematic side cross sectional view of the in-situ probe illustrated in FIG. 2 according to another embodiment of the present invention.

### DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The invention generally relates to an arrangement and/or method for monitoring an ongoing process, particularly a biological process. In many of its aspects, the invention relates to approaches for analyzing the contents of a bioreactor as a function of time, using, for example, an in-situ probe. Cells and/or substances can be identified and often quantified using a suitable technique. Furthermore, cells and/or other constituents can be detected, at various time intervals, and observed, e.g., in real time, as their concentration may fluctuate or as they are generated or consumed. Examples of processes that can be monitored include cell growth protocols, fermentations, and so forth.

Generally, techniques described herein are practiced with a flexible bioreactor, also known as a "rocking" or "bag" bioreactor. Some examples of commercially available rocking bioreactors include: BIOSTAT^{®} RM TX & Flexsafe^{®} RM TX Bags from Sartorius Stedim Biotech; ReadyToProcess WAVE^{™} 25 Bioreactor System from GE Healthcare; HyPerforma Rocker Bioreactor from ThermoFisher Scientific; and others. Rocking bioreactors can accommodate various process volumes (from microliters to many liters) and support various operations including, for instance, media preparation, cell cultivation, process development or optimization, microbial processes, etc.

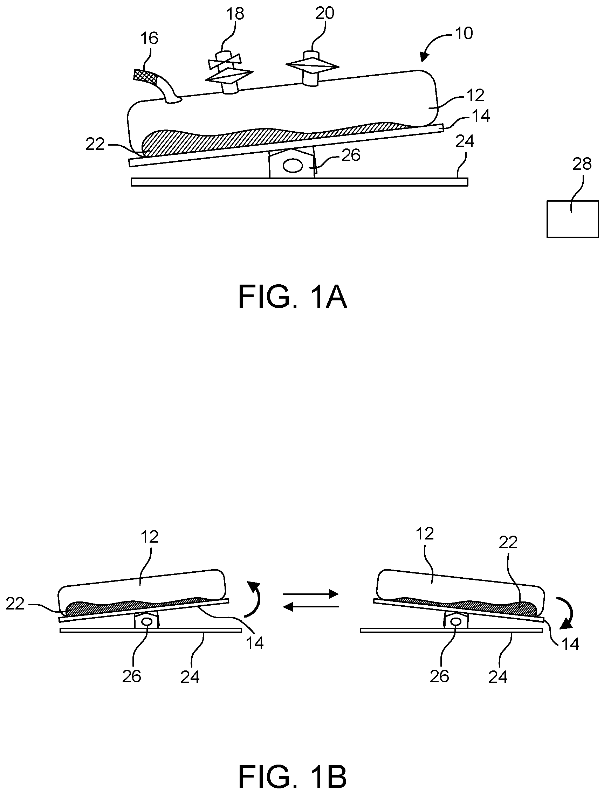

In a typical rocking reactor, such as rocking bioreactor 10 in FIGS. 1A and 1B, the process is conducted in a flexible bag 12 disposed onto rocking plate 14, which can be heated. Bag 12 can be provided with access ports (see ports 16, 18 and 20 in FIG. 1A) that are typically fused to the bag wall. Ports to a rocking bioreactor can be used to load ingredients, provide aeration (at a rate of from about 10 to about 500 ml/minute, for example), provide end-of-run drainage, and so forth. In some designs, multiple (two or more) bags can be rocked on a single rocking platform as illustrated in the rocking bioreactors shown at www.gelifesciences.com/en/us/shop/cell-culture-and-fermentation/rocking-bioreactors/systems/readytoprocess-wave-25-rocker-p-05542 and at www.sartorius.com/us-en/products/fermentation-bioreactors/single-use-bioreactors/biostat-rm-flexsafe-rm.

In many implementations, bag 12 is a flexible, single use (disposable) container that can be pre-sterilized. It can be provided in a collapsed state and inflated in place, using suitable instrumentation. Many designs involve a multi-layer (or "multi-film") arrangement.

Typically, the bags are made of USP (U.S. Pharmacopeia) Class VI plastics (resins certified for medical applications).

In one example, an outer layer, offering mechanical stability, is made from a semi-rigid thermoset such as polyethylene terephthalate or low density polyethylene (LDPE). A second layer, often made of polyvinyl acetate (PVA) or polyvinyl chloride (PVC), controls gas transfer. The interior or "contact" layer is made of PVA or polypropylene (PP).

In another example, the bag is made from multilayered USP Class VI plastics having a contact surface which is an ethylene-vinyl acetate (EVA)/LDPE copolymer and an outer layer designed to provide flexibility, strength, and extremely low gas permeability.

Generally, rocking bioreactors do not employ stirring devices inside the bag. Rather, mixing and gas transfer are promoted by waves established in fluid 22, e.g., a cell culture, contained in bag 12. The waves are induced by a rocking motion (as illustrated in FIG. 1B), often a gentle, low sheer rocking motion, provided, for instance, by a motorized base 24 which includes pivot axle 26. In many configurations, the motorized base is adjustable with respect to the rocking speed (e.g., within the range of from about 5 to about 40 rocks per minute) and angle (e.g., withing the range of from about 5 to about 10 degrees). For cell cultures, the rocking motion also can prevent cells from settling.

Culture conditions such as dissolved oxygen, temperature, and pH can be monitored. Automated systems, using controller 28, for example, streamline operations and minimize the need for manual interventions.

In one implementation, the bioreactor houses or is a cell culture system for the three-dimensional assembly, growth and differentiation of cells and tissues. In other implementations, the bioreactor contains cells, culture media, nutrients, metabolites, enzymes, hormones, cytokines and so forth.

To monitor the presence and/or concentration of ingredients on an ongoing basis, as the process unfolds, a rocking bioreactor such as shown in FIGS. 1A-1B is provided with an in-situ analysis probe, e.g., for analyzing at least some of these constituents using a system for determining their spectral response. Analysis can be in one or more of the following electromagnetic spectral regions: millimeter, microwave, terahertz, infrared (including near-, mid- and/or far- infrared), visible, ultraviolet (UV), x-rays and/or gamma rays. Further, the spectroscopy system can measure different characteristics, such as absorption spectra, emission (including blackbody or fluorescence) spectra, elastic scattering and reflection spectra, impedance (e.g., index of refraction) spectra, and/or inelastic scattering (e.g., Raman and Compton scattering) spectra, of analytes in the bioreactor.

Illustrative embodiments described herein rely on spectroscopy in the ultraviolet, visible regions, or the infrared region, e.g., extending from 700 nanometers (nm) to 1 millimeter (mm) in wavelength and specifically including the near infrared (0.75-1.4 microns (µm), NIR), short-wavelength infrared (1.4-3 µm, SWIR), mid-wavelength infrared (3-8 µm, MWIR), long-wavelength infrared (8-15 µm, LWIR), and the far infrared (15-1000 µm, FIR) of the spectrum. In many embodiments, the techniques employed to detect cells or other process ingredients such as, for example, culture media, nutrients, metabolites, enzymes, hormones, cytokines and so forth, rely on NIR spectroscopy and, in many cases, NIR-SWIR spectroscopy. Probing molecular overtone and combination vibrations, NIR-SWIR spectroscopy covers the region of from 780 nanometer (nm) to 2500 nm of the electromagnetic spectrum.

An overview of NIR spectroscopy can be found, for example, in an article by A.M.C. Davies in "An Introduction to Near Infrared (NIR) Spectroscopy", www.impublications.com/content/introduction-near-infrared-nir-spectroscopy. See also, Cervera, A. E., Petersen, N., Lantz, A. E., Larsen, A. & Gernaey, K. V. Application of near-infrared spectroscopy for monitoring and control of cell culture and fermentation, Biotechnol. Prog. 25, 1561-1581 (2009); and Roggo Y, et al., "A review of near infrared spectroscopy and chemometrics in pharmaceutical technologies", Journal of Pharmaceutical and Biomedical Analysis, Volume 44, Issue 3, 2007.

Among its strength, infrared spectroscopy presents a non-invasive, non-destructive investigative approach, typically involving fast scan times.

According to embodiments described herein, measurements are taken within (inside) the bag of the bioreactor, typically without a need to withdraw a sample from the reactor and direct it to a sample cell or to an external (ex-situ) arrangement for taking a reading.

The frequency of measurement can be set as desired, about every 5 minutes, for instance. In many implementations, the sampling is repeated with a desired frequency over any desired time period. For example, sampling is repeated (e.g., at a few minute-intervals) to monitor the entire reactor process (e.g., for a week, two weeks, three weeks or longer).

Once a desired scan rate is set, the scanning program can be started.

Aspects of the invention feature approaches that employ a probe for monitoring a process conducted in a flexible bag of a rocking bioreactor such as the one depicted in Figs. 1A and 1B.

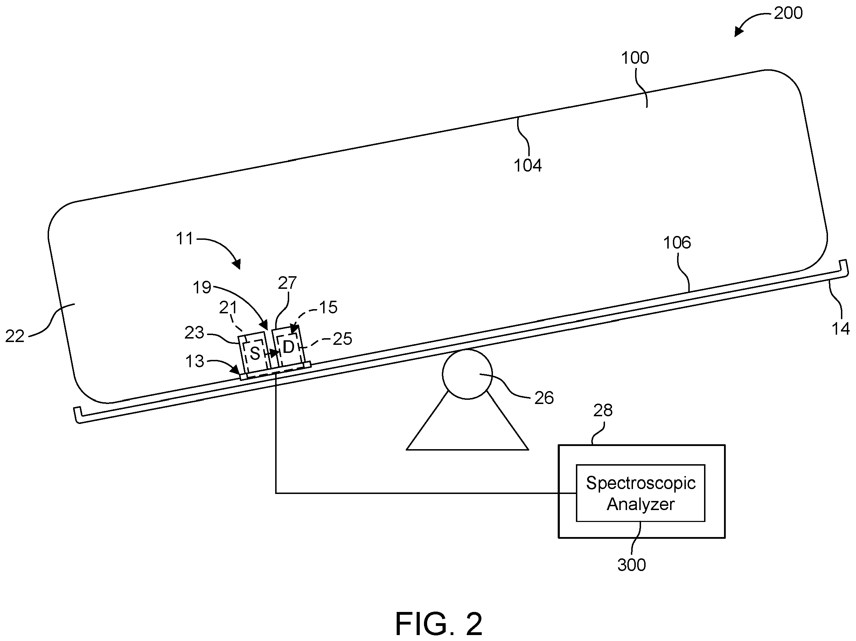

FIG. 2 is a view of a rocking bioreactor 200 including a bag 100 disposed onto a rocking platform 14 and fitted with an in-situ probe 11, according to embodiments of the present invention.

The in-situ probe 11 comprises a patch 13 and a probe module 15.

The patch 13 is often fused or otherwise bonded or secured to bottom or side wall 106 of the bag 100 (e.g., via welding, thermo-forming, or adhesive), defining a sample gap or sample spacing 19, which, for example, is filled by a sample of the bag's contents 22. The patch 13 is often disposable, along with the bag 100.

The probe module 15 includes one or more source elements 21, housed in source assembly 23, one or more detector elements 25, housed in detector assembly 27, and often a bench or common assembly base. The source element(s) 21 emit light which propagates through the sample gap, and the detector element(s) 25 receive the light for detection after interaction with the contents of the bag. In one example, the probe module 15 is a detachable and in many cases reusable part, insert or component that can be mated to the patch, then disassembled and typically reused.

Bioreactor 200 can be controlled, e.g., in automated fashion, using a controller such as controller 28 in FIG. 1A. The same or a different controller can be employed to conduct the NIR analysis. In the illustrated example, the controller 28 includes a spectroscopic analyzer 300 that conducts the spectroscopic analysis. The spectroscopic analyzer 300 includes a single board computer in one example. It monitors the response of the detector elements 25 (e.g., photodiode(s)) as a function of the instantaneous wavelength of the tunable laser (directed from the source element(s) 21 through the sample gap) in order to resolve the absorption spectrum. of the material in the sample detection area.

In more detail, in operation according to one embodiment, the spectroscopic analyzer 300 comprises a narrow band tunable light source such as a tunable laser to interrogate specific wavelengths or wavelength bands of electromagnetic spectrum. to perform absorption spectroscopy on the contents of the bag 100. In one example, the laser source spectrally sweeps through a portion of a wavelength band. This band can be in the ultraviolet or visible or the infrared, which extends from 700 nanometer (nm) to 1 millimeter of the electromagnetic spectrum. In one embodiment, this light is supplied to the probe 11, and the laser's emission is formed into a beam by a collimator and molded aspheric lens to propagate across the sample gap 19 and then be detected by a detector element 25, e.g., a photodiode.

In other examples, a broadband source could be used in conjunction with a spectrally resolving detector.

Configurations such as described above also can be employed in multiplexed experiments, where the rocking plate (e.g., rocking plate 14 in FIGS. 1A and 1B) can support two or more bags 100, each bag having its own in-situ probe, for example. With cell cultures or fermentation processes, multiplexed experiments, using, for instance micro- or mini-bioreactors, make possible the evaluation of process conditions, cell lines, or other variables in an efficient manner and may find applications in scale-up work.

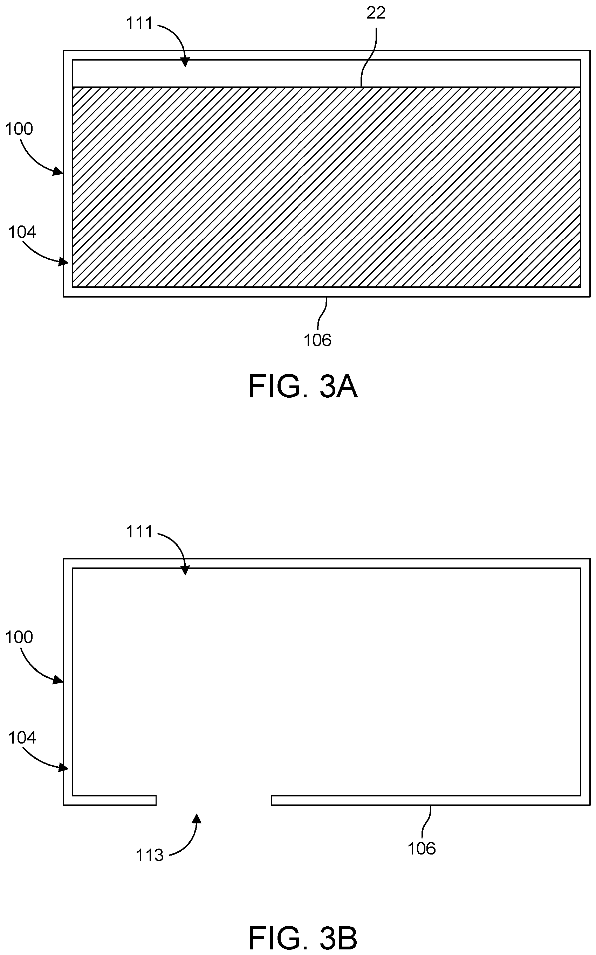

FIG. 3A shows an exemplary bag 100 to which the present invention is applicable. Walls 104 of the bag define a volumetric region 111 that contains reaction material. When installing the patch, a hole (opening) 113 is formed in the wall of the bag, as illustrated in FIG. 3B, or the bag is directly fabricated with the hole. In the illustrated example, the hole is formed in a bottom wall 106 of the bag.

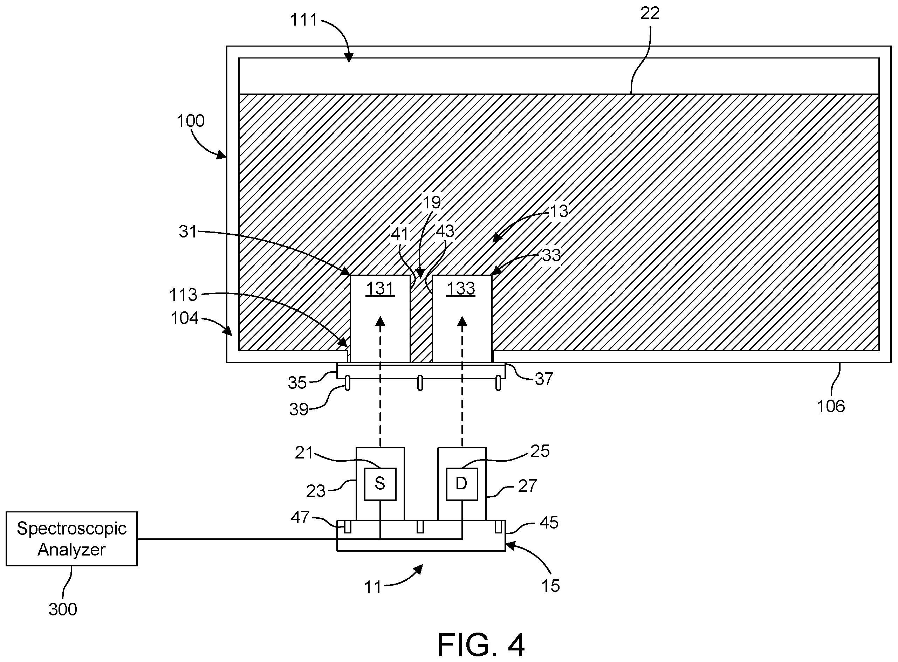

FIG. 4 shows an example of the probe illustrated in Fig. 2 according to one embodiment of the present invention.

As before, probe 11 comprises the patch 13 and the probe module 15.

The patch 13 comprises a source hood 31, a detector hood 33, and a patch rim 35 that are typically formed from a unitary piece of often transparent plastic or another material such as glass.

The patch 13 is inserted into the hole 113 in the bottom wall 106 of the bag such that the source hood 31 and the detector hood 33 project inward from an outer wall 106 of the bottom portion of the bag, forming a source chamber 131 and a detector chamber 133 within the bag's outer perimeter. The chambers formed by the hoods are accessible from outside the bag and contain, for example, ambient air from an environment surrounding the bioreactor. The chambers displace the contents 22 of the bag (e.g., a liquid solution).

The patch 13 is secured to the wall of the bag via a patch rim 35 that is bonded, such as by welding or cement, to an inner or outer wall of the bag to form a seal over the bottom hole 113. A patch weld seam 37 between the patch rim and the bottom wall of the bag extends around a perimeter of the hole 113. The patch rim often comprises a fiducial arrangement such as one or more alignment pegs 39, which project outward from an exterior surface of the patch rim 35. In one example, the alignment pegs are integral with the patch rim.

The patch defines a sample gap in the interior of the bag. In the illustrated example, between the chambers 131 and 133, defined, respectively, by the source hood 31 and the detector hood 33 of the patch 13 is the sample gap 19, which is a space, which, during normal operation of the bioreactor would be filled by a sample of the bag's contents.

In specific embodiments, the source hood 31 comprises a source window portion 41, and the detector hood 33 comprises a detector window portion 43. The window portions face each other and define the lateral sides of the sample gap 19.

The probe module 15 comprises a source assembly 23, a detector assembly 27, one or more source elements 21, one or more detector elements 25, a common assembly base (or bench) 45, and alignment holes 47. These last elements are holes bored into an exterior surface of the common assembly base that contacts an exterior surface of the patch rim 35 when the probe module 15 and the patch rim are mated or fully assembled.

The source assembly 23, housing at least one of the one or more source elements 21 inserts into the source hood 31 (e.g., into the chamber 131 defined by the source hood).

Likewise, the detector assembly 27, housing at least one of the one or more detector elements 25, inserts into the detector hood 33 (e.g., into the chamber 133 defined by the detector hood).

In the illustrated example, the source assembly and the detector assembly are secured to or integral with the common assembly base 45, which, in different embodiments, can house additional source and/or detector elements as well as cables and/or other connectors for forming electrical and/or fiber optic connections between the source and detector elements and the spectroscopic analyzer 300.

In general, physical attributes (e.g. size, shape, relative position or spacing with respect to each other) of the source assembly and detector assembly are based on analogous physical attributes of the source hood and the detector hood of the patch, such that the probe module is insertable into the patch, with the source hood 31 receiving the source assembly 23 and the detector hood 33 receiving the detector assembly 27.

In the illustrated example, the probe is in a disassembled state, as the probe module 15 has not been mated with or inserted into the patch 13.

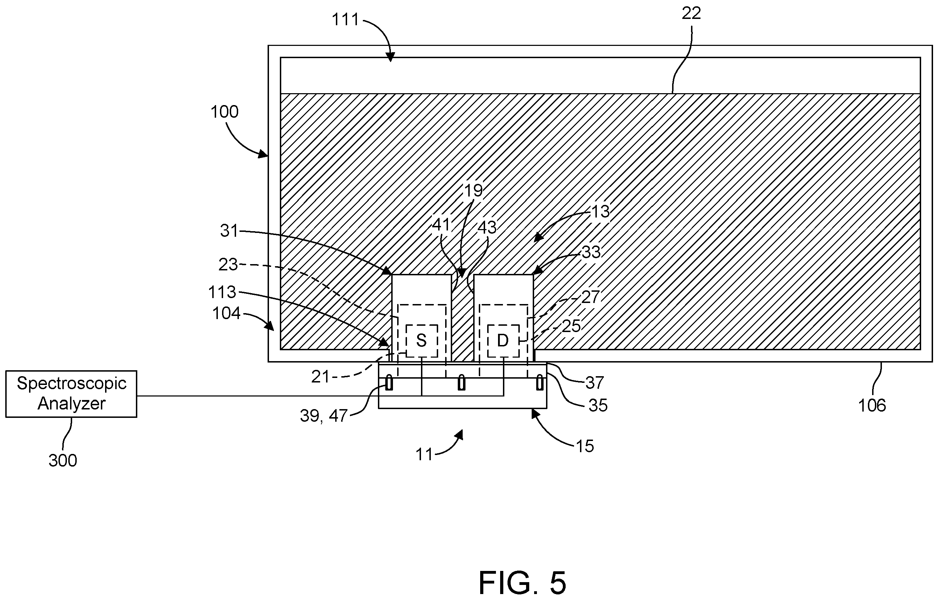

FIG. 5 shows the probe illustrated in Fig. 4 with the probe fully assembled (e.g., the probe module 15 is mated with or inserted into the patch 13). Now, the source assembly 23 and the detector assembly 27 are within the respective chambers 131 and 133, defined by the source hood 31 and the detector hood 33 of the patch. Like the source hood and the detector hood, the source assembly and the detector assembly project inward from the outer perimeter of the bag and from the patch rim 35. The alignment pegs 39 of the patch insert into the alignment holes 47 of the common assembly base of the probe module 15.

In this way, the source element(s) 2 1 housed by the source assembly 23 are configured to emit or reflect light through the source window portion 41 of the source hood 31, across the sample gap 19, through the detector window portion 43 of the detector hood 33, and to the detector element(s) 25 housed by the detector assembly 27. Thus, the contents 22 of the bag 100 can be spectroscopically analyzed (by spectroscopic analyzer 300) without breaching the sterile field of the bag's interior.

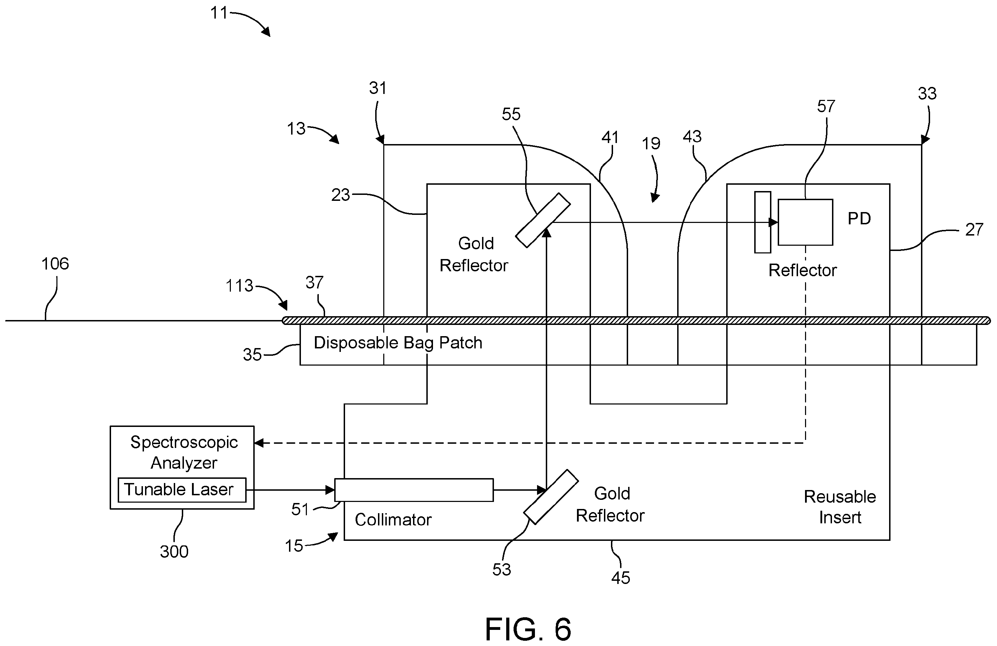

FIG. 6 is a diagram of the in-situ probe illustrated in Fig. 2 according to one embodiment of the invention.

The probe comprises the patch 13 and the probe module 15. As previously described, the patch defines a sample gap 19 between the source hood and the detector hood.

The probe module 15 comprises the source element(s) 21 and the detector element(s) 25 and inserts into or mates with the patch 13, with the source hood 31 of the patch receiving the source assembly 23 of the probe module and the detector hood 33 of the patch 13 receiving the detector assembly 25 of the probe module. The source and detector assemblies are attached to or integral with the common assembly base or bench 45.

This embodiment of the probe module comprises several source elements, namely a collimator 51, a first fold mirror (gold reflector substrate) 53, a second fold mirror/ gold reflector, and a detector element, namely photo detector 57.

The collimator 51 is a source element that receives light (e.g. from a tunable laser 59 via a fiber optic cable or via freespace transmission) and forms the light into a beam (solid line arrow), which is directed via other source elements, including the first and second mirrors or reflectors (53, 55), out of the chamber defined by the source hood, through the sample gap 19, into the chamber defined by the detector hood, and to the photodetector, which is a detector element that outputs (dashed line arrow) to the spectroscopic analyzer 300 an electrical signal indicative of properties of the light after interaction with the contents of the bag.

In the illustrated example, the common assembly base 45 houses the collimator 51 and the first mirror (reflector) 53, which directs the beam from the collimator to the second mirror (reflector) 55, which is housed by the source assembly 23 and positioned within the chamber defined by the source hood 31 such that it receives the light from the first reflector and directs the light through the source window portion 41, into the sample gap 19. After passing through the sample gap, light is transmitted through detector window portion 43, and then reaches photodetector 57 which is housed by the detector assembly 27 within the chamber defined by the detector hood 33 and positioned to receive and detect the light transmitted from the second reflector 55.

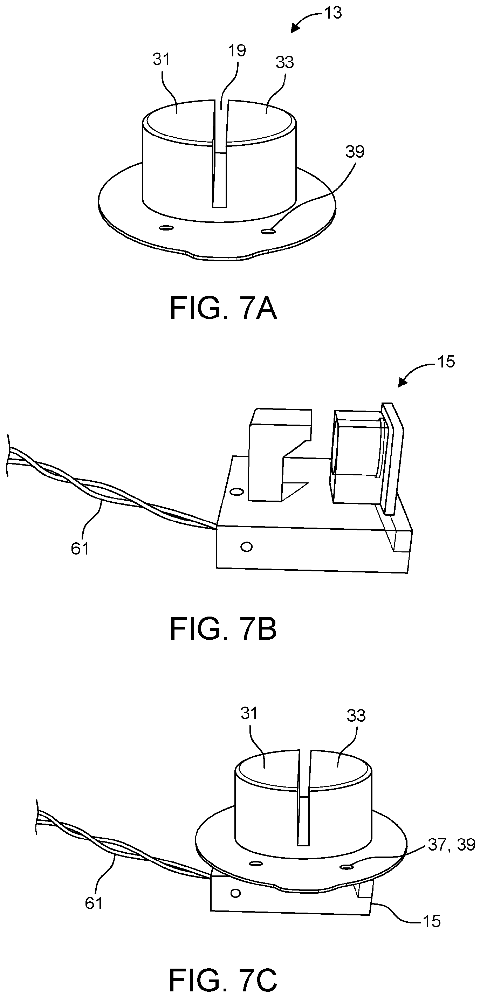

In general, FIGS. 7A, 7B, and 7C are perspective views of an exemplary in-situ probe 11.

More specifically, FIG. 7A shows the patch 13 with the source hood 31 and detector hood 33, which define the sample gap 19 between the source and detector hoods. The patch is preferably formed from transparent or translucent material allowing light from the source to transmit through the patch. In one example, the patch is injection molded and formed of polycarbonate plastic. In other examples, polypropylene or other materials might be used, and/or the source and detector window portions (41, 43) of the patch include transparent slides (e.g., plates) that form an interface between the source element(s) 21 housed in the source assembly 23 and the detector element(s) 25 housed in the detector assembly 27, when the probe module is mated with the patch. The patch comprises the alignment pegs for positioning the patch on the wall of the bag and/or aligning the probe module with the patch 13. In the illustrated example, the patch has a height of 12 millimeters (mm). Typically, the height will be between 5 and 50

FIG. 7B shows the probe module 15, which inserts into or mates with the patch 13 (of Fig. 7A). In the illustrated example, the probe module 15 has a base protrusion of around 4 mm. Electrical wires and possibly fiber optic cabling 61 connect the probe module with the controller and/or tunable laser.

FIG. 7C shows the full assembly of the in-situ probe 11, with the probe module 15 inserted into or mated with the patch 13.

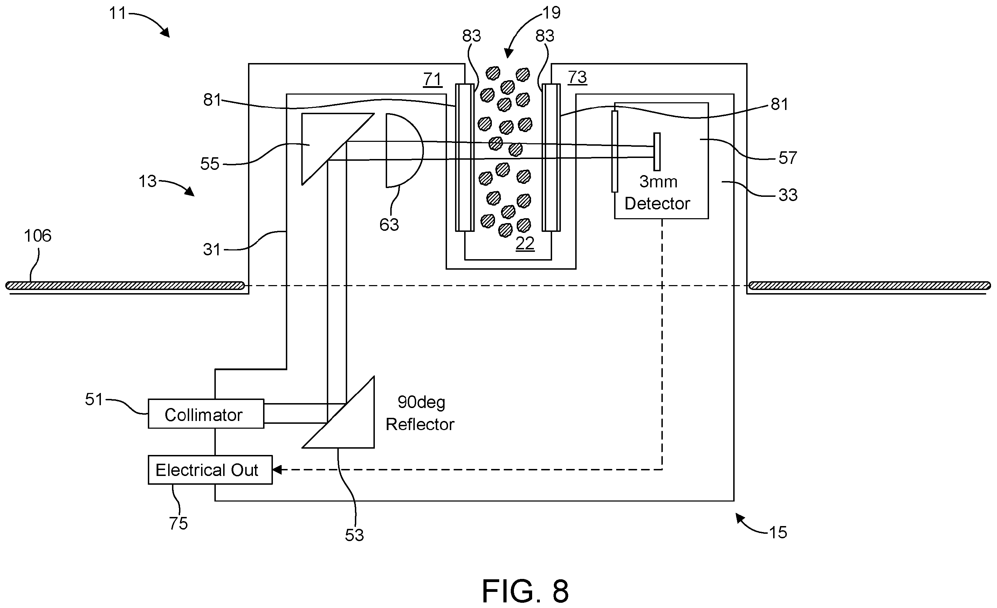

FIG. 8 is a diagram of the in-situ probe 11 illustrated in Fig. 2 according to another embodiment of the invention.

The probe comprises the patch 13 and the probe module 15 inserted into or otherwise mated with the patch.

As previously described, the patch defines a sample gap 19 between the source hood 31 and the detector hood 33, and contents of the bag (e.g. a liquid solution including cells) fill the sample gap.

Here, the probe module comprises the collimator 51, first and second reflectors (53, 55), a lens 63, a first window 71, a second window 73, and the photodetector 57.

As before, the collimator 51 receives light (e.g., from a tunable laser via a fiber optic cable) and forms the light into a beam with low divergence. This light is directed via the first and second reflectors, reflectors 53 and 55, respectively, out of the chamber defined by the source hood 31, across the sample gap 19, into the chamber defined by the detector hood 33, and to the photodetector 57, which outputs (dashed line arrow) to the spectroscopic analyzer 300, via an electrical output port 75, for example, an electrical signal indicative of properties of the light after interaction with the contents of the bag.

In the illustrated example, the second reflector directs the light through a focusing lens 63, which is one of the one or more source elements 21 housed by the source assembly 23, and through the first window 71 and the second window 73, which are secured to or form walls of the source and detector hoods 31 and 33 (respectively) as the source and detector window portions that define the sample gap. The first and second windows form an optical interface between the chamber defined by the source hood 31 and the chamber defined by the detector hood 33. In one example, interior surfaces of the windows (i.e., the surfaces in contact with air within the chambers 131 and 133) have an anti-reflective coating 81, and the exterior surfaces of the windows (i.e., the surfaces in contact with the contents of the bag) have an index-matched coating 83 to provide a refractive index matched to or based on the contents of the bag. Some implementations of the probe can employ only one window. In one example, one of the hoods retains the window portion described above, while the other hood is provided with a window.

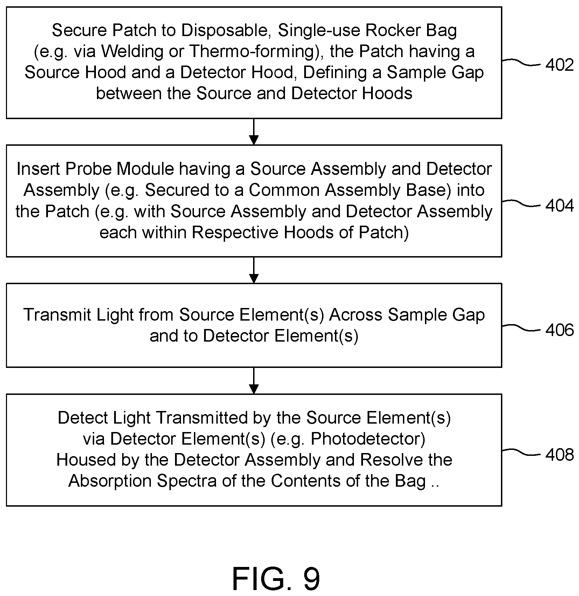

FIG. 9 is a flow diagram illustrating the process by which the in-situ probe 11 is used to monitor a process within a bag.

First, the patch is secured to a wall of the bag (e.g., step 402 relates to installing the patch into a disposable, single-use rocker bag via welding or thermo-forming). The patch defines a sample gap between the source hood and the detector hood of the patch.

In step 404, a probe module with one or more source elements and one or more detector elements housed, respectively, in a source assembly and detector assembly, the probe module being secured to or integral with a common base is then inserted into the patch, for example, with the source assembly and detector assembly being received by the respective chambers defined by the source and detector hoods of the patch.

According to step 406, the source element(s) transmit(s) light through and across the sample gap to the detector element(s) under the control of the spectroscopic analyzer 300 of the controller 28.

Finally (step 408), one of the detector elements, namely the photodetector, detects the light after interaction with the contents of the bag, and the absorption spectra of the contents of the bag are resolved and analyzed by the spectroscopic analyzer 300 to determine attributes of the bag contents.

When a tunable laser is used, the spectroscopic analyzer 300 monitors the response of the photodiode 57. Thus, the analyzer resolves the absorption spectra of the sample by monitoring the spectral scanning of the tunable laser over its scan band relative to the time-response of the photodiode 57. Generally, the tunable laser or tunable laser system sweeps its narrow band emission over some region of the electromagnetic spectrum such as the NIR and/or SWIR regions, or portions thereof.

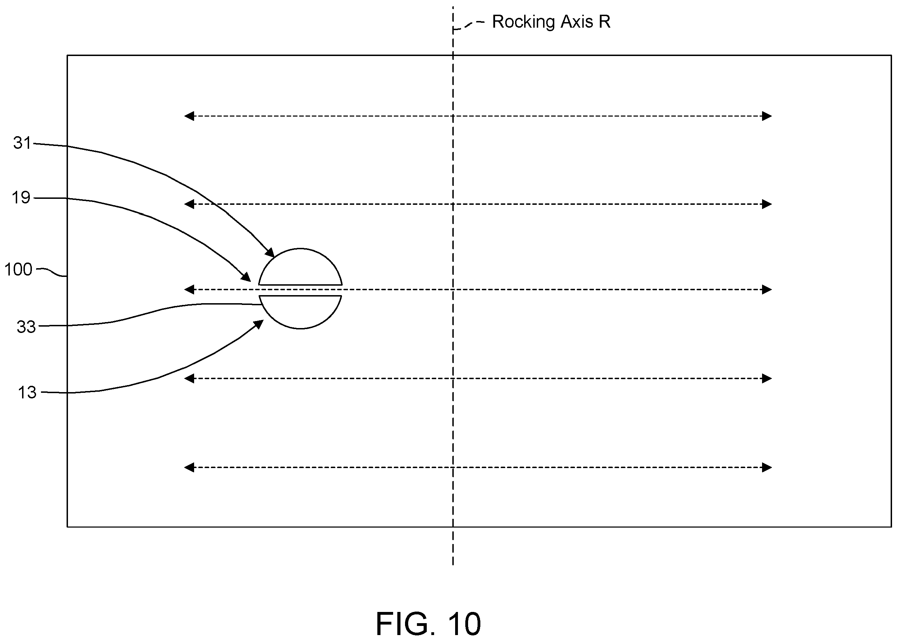

Fig. 10 is a plan view of the in-situ probe showing how the patch can be advantageously positioned based on the direction of fluid motion in a rocking bioreactor bag 100. In the illustrated example, the bioreactor rocks along a rocking axis R, causing liquid contained by the bag to move transverse to the rocking axis in both directions (e.g., both left and right as illustrated, across the rocking axis R), assuming a dominant direction of fluid motion indicated by six dashed-line arrows. The patch is positioned such that the sample gap 19 forms a channel, the extent of which is transverse to the rocking axis and parallel to the dominant direction of fluid motion, allowing tidal action of the liquid to wash new fluid into and out of the sample gap, preventing stagnation and increasing accuracy of the probe. Other arrangements are possible.

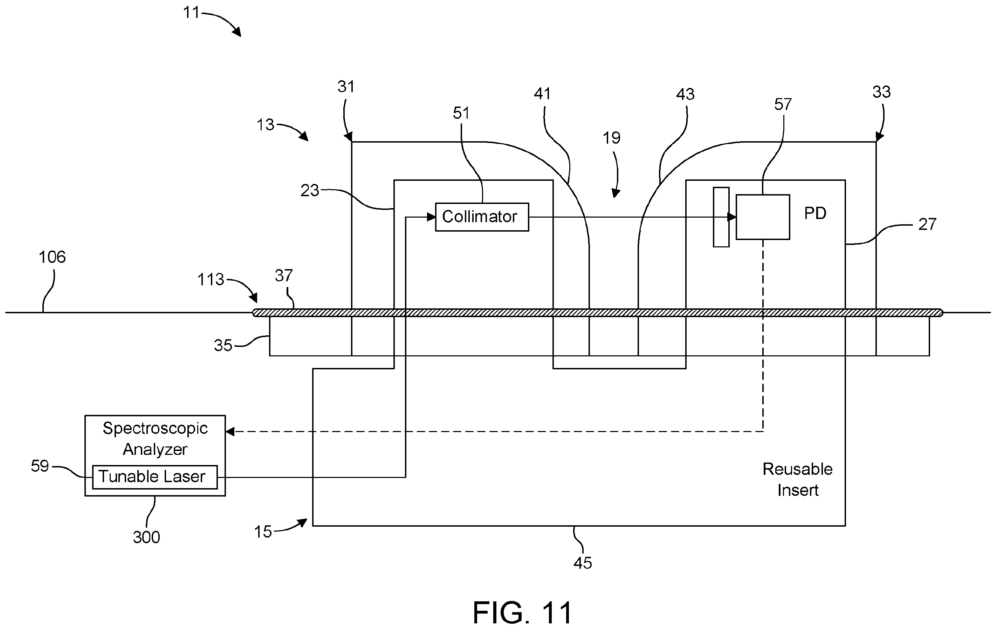

FIG. 11 is a diagram of the in-situ probe 11 illustrated in FIG. 2 according to a further embodiment of the invention. The probe includes the probe module 15 (which can form a reusable insert) fitted into the patch 13, as previously described with respect to FIG. 3. Now, however, the collimator 51 is housed by the source assembly 23 of the probe module 15, receiving the light from the tunable laser 59, in the direction of the arrow (solid line), via a fiber optic cable, for example, and guiding the light into a beam directly to the photodetector 57, housed by the detector assembly 27, without the use of intervening reflectors.

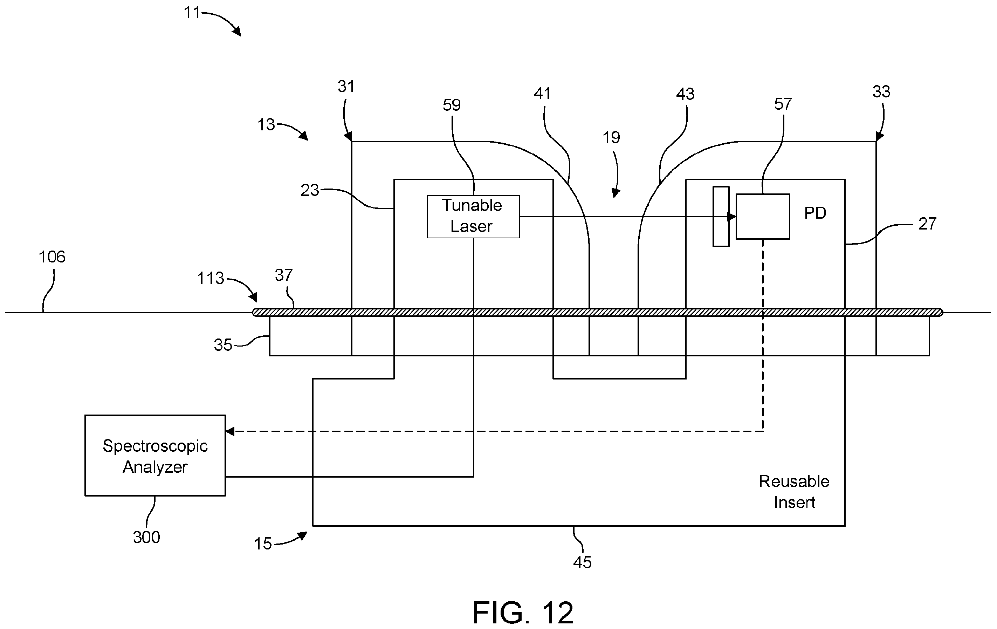

Likewise, FIG. 12 is a diagram of the in-situ probe 11 illustrated in FIG. 2 according to another embodiment of the invention. The probe includes the probe module 15, reusable, for example, inserted into the patch 13 as previously described with respect to FIG. 3. Now, however, the tunable laser 59 is housed by the source assembly 23 of the probe module, emitting the light (which propagates through sample gap 19) directly to the photodetector housed by the detector assembly 27, without the use of intervening reflector(s) and/or collimator.

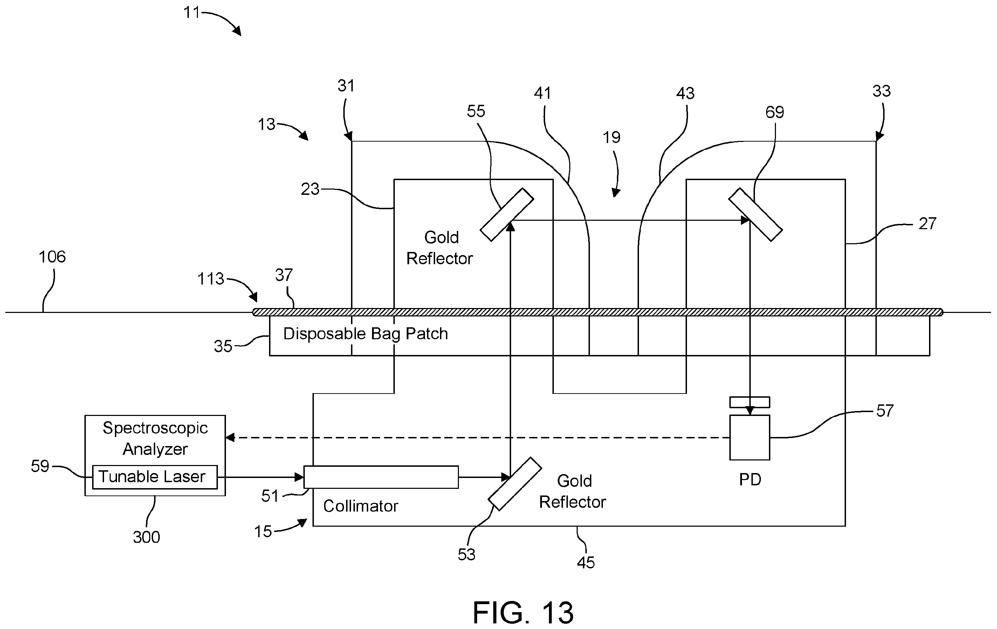

FIG. 13 is a diagram of the in-situ probe 11 illustrated in FIG. 2 according to yet another embodiment of the invention. The probe includes the probe module 15 (reusable in many cases) inserted into the patch 13 (typically disposable, e.g., with the bag) as previously described with respect to FIG. 3. Now, however, the photodetector 57 is housed in the common assembly base 45 of the probe module 15 rather than in the detector assembly; a third reflector 69, which is a detector element housed by the detector assembly 27, directs (solid line arrow) the light received from the source element(s), e.g., collimator 51, first reflector (mirror) 53, second reflector (mirror) 55, back to the photodetector 57 housed by the common assembly base 45.

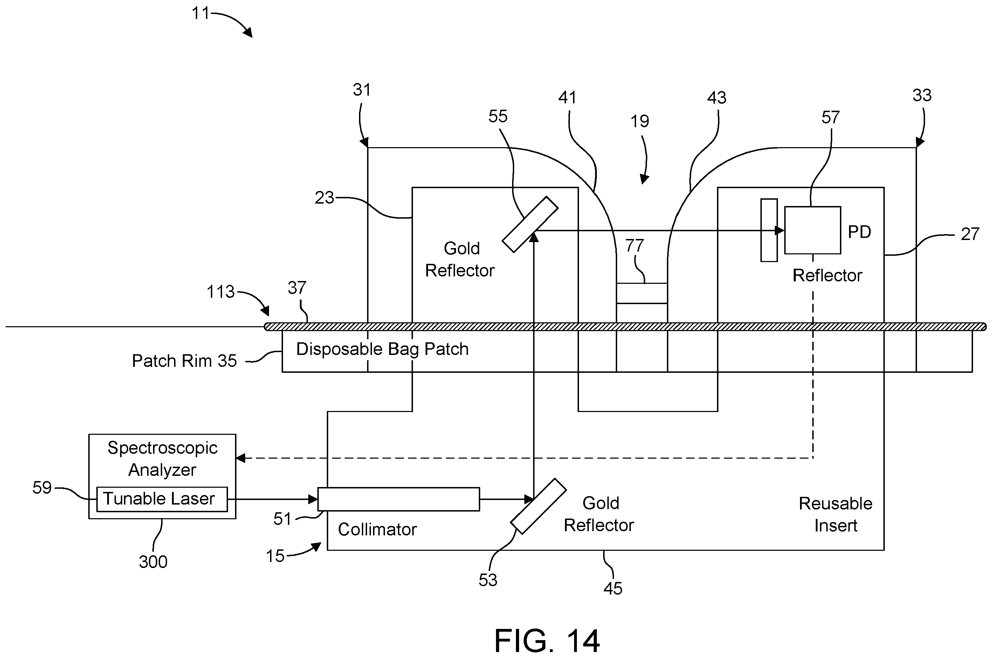

FIG. 14 is a diagram of the in-situ probe illustrated in FIG. 2 according to still another embodiment of the invention. Here, a mechanical spacer 77 ensures a fixed, uniform, predetermined spacing between the source window portion 41 and the detector window portion 43. In one example, the patch is formed of non-rigid plastic, and the mechanical spacer provides the fixed distance between the source hood and detector hood. On the other hand, the fixed distance between the hoods can also be provided by forming the patch from a rigid material, thus maintaining a fixed gap pathlength, among other examples.

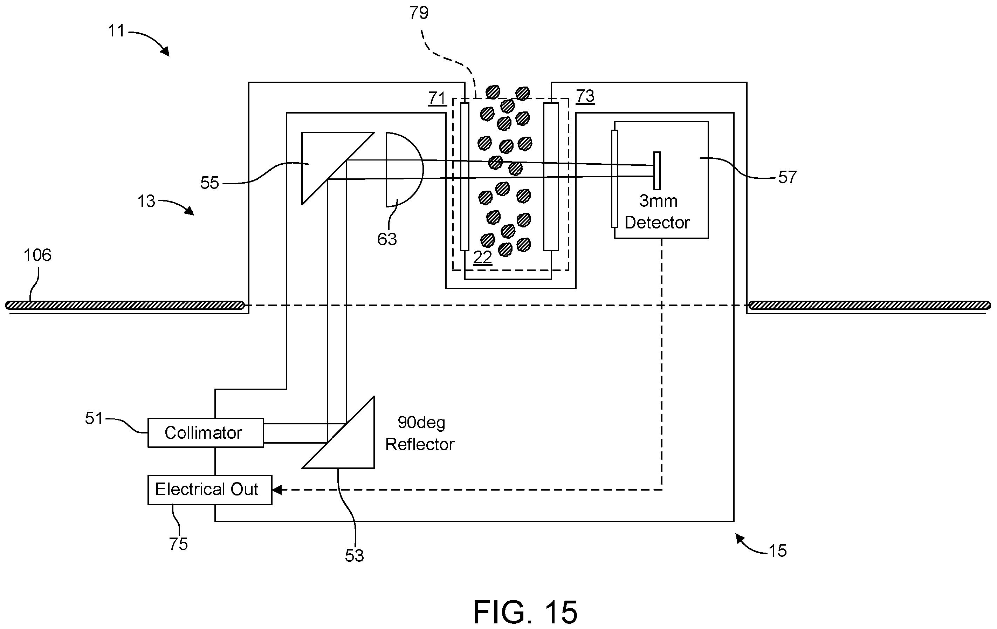

FIG. 15 is a diagram of the in-situ probe illustrated in FIG. 2 according to another embodiment of the invention. The probe includes the probe module 15 inserted into the patch 13, as previously described with respect to FIG. 3, and the first window 71 and second windows 73 providing the optical interface between the source element(s) 21, namely collimator 51, mirror 53, mirror 55, lens 63), and the detector element(s) housed by the detector assembly, photodetector 57, for example. Now, however, the first window 71 has a different thickness than the second window 73, forming an etalon 79. The etalon traps certain wavelengths of light, allowing the probe to be used to capture changes in refractive index such as due to protein aggregation within the bag, for example, or the purposes of determining viability of cells within the bag.

More specifically, having an index of refraction that is different from that of the culture medium, as in the etalon depicted in FIG. 15, causes some of the light to become scattered or refracted in the sample gap, reducing the contrast of the etalon window and thus the signal obtained by the photodetector. The scattering produced by the viable cells is exhibited in a lowering of the contrast of the window etalon.

In practice, live cells in the culture medium are expected to change the Fast Fourier Transform (FFT) signal for the light relative to a matched culture medium that is free of live cells. (As known in the art, FFT processing relates to a technique based on an algorithm that computes the discrete Fourier transform (DFT) of a sequence, or its inverse (IDFT)). In addition, the magnitude of the peak observed with respect to the length scale will reflect changes in cell viability. For example, a peak will increase as some cells undergo autolysis, since those cells are expected to approach the index of refraction of the culture medium and fewer cells with lower scattering will be encountered by the spherical wave-front.

According to this general approach, described by Hassell et al. in U.S. Patent Application No. 17/094,901, with the title Fabry Perot Interferometry for Measuring Cell Viability, filed on November 11, 2020, published on May 13, 2021 as U.S. Patent Application Publication No. 2021/0140881 A1, the life cycle of cells is generally marked by various changes. For instance, before autolysis (i.e., the destruction of cells or tissues by their own enzymes, such as enzymes released by lysosomes) cells will typically stain a deep red. As autolysis progresses, the staining becomes gradually fainter, probably due to losses in stainable material, and the cells appear disorganized. In addition, cells undergoing autolysis have an index of refraction that approaches the index of refraction of the aqueous cell culture, possible due to a loss in cell density and/or other mechanisms. In contrast, live cells have an index of refraction that is different from that of the aqueous culture medium, resulting in a turbid environment.

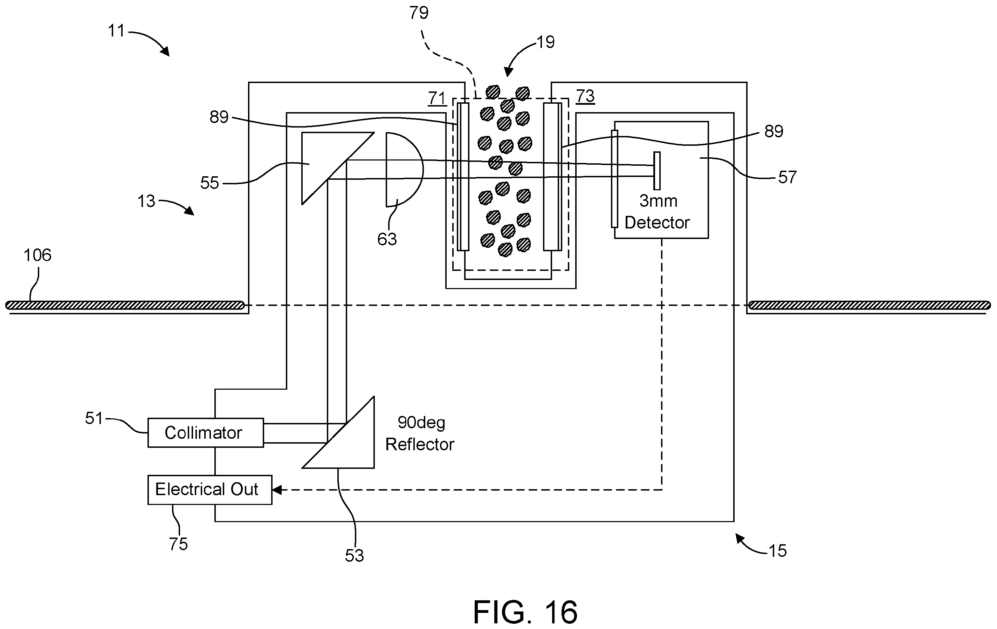

Fig. 16 is a diagram of the in-situ probe illustrated in Fig. 15 according to another embodiment of the invention. As before, two windows (71 and 73) of different thicknesses form an etalon 79 from the source window portion 41, across the sample gap 19, to the detector window portion 43. Now, however, the interior surfaces of the windows (e.g., the surfaces in contact with air within the source and detector chambers) have a partially reflective coating 89.

In still other examples, the photodetector 57 is replaced with a spatially resolved image detector such as a silicon CCD or CMOS image sensor or an InGaAs spatially resolved sensor. Preferably these sensors includes a two dimensional pixel array of more than 100 pixels along each axis. This allows for the detection of images and detection of diffraction patterns.

While this invention has been particularly shown and described with references to preferred embodiments thereof, it will be understood by those skilled in the art that various changes in form and details may be made therein within the scope of the invention encompassed by the appended claims.

## Claims

1. A system (11) for monitoring a process conducted within a bag (100,12) for a bioreactor, the system comprising:
a patch (13) configured to be secured to a wall (106) of the bag and defining a sample gap (19) in the interior of the bag, wherein the patch (13) comprises
at least one hood that projects into the bag; and
a probe module (15), configured to be inserted into or to mate with the patch (13), for monitoring contents (22) of the bag, the probe module (15) comprising a source element (21) and a detector element (25) for receiving light transmitted from the source element (21) and through the sample gap (19) after interaction with the contents (22).

2. The system as claimed in claim 1, wherein the bag (100,12) is a flexible bag of a rocking bioreactor (10).

3. The system as claimed in claim 1, wherein the patch (13) comprises a source hood (31) and a detector hood (33), which project inward from an outer wall (106) of the bag, a wall of the source hood and a wall of the detector hood defining the sample gap (19) therebetween.

4. The system as claimed in claim 3, wherein the source hood (31) and the detector hood (33) are each accessible from outside the bag.

5. The system as claimed in claim 4, wherein the probe module (15) inserts into the patch (13) such that the source hood (31) receives a source assembly (23) housing the source element (21) and the detector hood receives (33) a detector assembly (27) housing the detector element (25).

6. The system as claimed in claim 5, wherein the source assembly (23) and the detector assembly (27) are both secured to or integral with a common assembly base (45) of the probe module (15).

7. The system as claimed in claim 6, wherein sizes and shapes of the source assembly (23) and the detector assembly (27) and spacing between the source assembly and the detector assembly correspond to sizes and shapes of the source hood (31) and the detector hood (33) and the spacing therebetween.

8. The system as claimed in claim 1, wherein the patch (13) is configured to be secured to the wall (106) of the bag via welding, thermo-forming, or adhesive.

9. The system as claimed in claim 1, wherein the bag (100,12) and the patch (13) are disposable or single-use components, and the probe module (15) is reusable with different patches and bags.

10. The system as claimed in claim 1, wherein the patch comprises one or more window plates (41,43) for transmitting the light between the sample gap (19) and at least one of the source hood (31) and the detector hood (33).

11. A method for monitoring a process conducted within a bag (100,12) for a bioreactor using the system of claim 1, the method comprising:
securing the patch (13) to a wall (106) of the bag (100,12);
directing light from the source element (21) of the probe module (15) through the sample gap (19); and
detecting the light at the detector element (25) of the probe module (15) after interaction between the light and the contents (22) of the bag.

12. The method as claimed in claim 11, wherein the bag (100,12) is a flexible bag of a rocking bioreactor (10).

13. The method as claimed in claim 11, further comprising a source hood (31) of the patch (13) and a detector hood (33) of the patch projecting inward from an outer wall (106) of the bag (100,12), a wall of the source hood (31) and a wall of the detector hood (33) defining the sample gap (19) therebetween.

14. The method as claimed in claim 13, wherein the source hood (31) and the detector hood (33) are each accessible from outside the bag.

15. The method as claimed in claim 14, further comprising inserting the probe module (15) into the patch (13) such that the source hood (31) receives a source assembly (23) housing the source element (21) and the detector hood (33) receives a detector assembly (27) housing the detector element (25).

16. The method as claimed in claim 15, wherein the source assembly (23) and the detector assembly (27) are both secured to or integral with a common assembly base (45) of the probe module.

17. The method as claimed in claim 16, further comprising configuring sizes and shapes of the source assembly (23) and the detector assembly (27) and spacing between the source assembly and the detector assembly to correspond to sizes and shapes of the source hood (31) and the detector hood (33) and spacing therebetween.

18. The method as claimed in claim 11, further comprising securing the patch (13) to the wall (106) of the bag via welding, thermo-forming, or adhesive.

19. The method as claimed in claim 11, wherein the bag (100,12) and the patch (13) are disposable or single-use components, and the probe module is reusable with different patches and bags.

20. The method as claimed in claim 11, wherein the patch comprises one or more window plates (41,43) for transmitting the light between the sample gap (19) and at least one of the source hood (31) and the detector hood (33).

21. A bag (100,12) for a bioreactor, the bag comprising:
a patch (13) secured to a wall (106) of the bag, the patch (13) defining a sample gap (19) and including at least one hood that projects into the bag, wherein a probe module (15) for monitoring contents (22) of the bag, and comprising a source element (31) and a detector element (33) for detecting light transmitted from the source element (31) through the sample gap (19) after interaction with the contents (22), can be inserted into or mated with the patch (13).

22. A rocking bioreactor system (10), comprising:
a motorized base (24);
a rocking bioreactor bag (100,12) according to claim 21.

## Patentansprüche

1. System (11) zum Überwachen eines Prozesses, der innerhalb eines Beutels (100, 12) für einen Bioreaktor durchgeführt wird, wobei das System aufweist:
ein Ansatzteil (13), das dafür ausgelegt ist, an einer Wand (106) des Beutels befestigt zu werden und einen Probenspalt (19) im Inneren des Beutels bildet, wobei das Ansatzteil (13) mindestens eine Haube aufweist, die in den Beutel hineinragt; und
ein Sondenmodul (15), das dafür ausgelegt ist, in das Ansatzteil (13) eingeführt oder mit diesem gekoppelt zu werden, zum Überwachen eines Inhalts (22) des Beutels, wobei das Sondenmodul (15) ein Quellenelement (21) und ein Detektorelement (25) zum Empfangen von Licht aufweist, das nach Wechselwirkung mit dem Inhalt (22) vom Quellenelement (21) durch den Probenspalt (19) übertragen wurde.

2. System nach Anspruch 1, wobei der Beutel (100, 12) ein flexibler Beutel eines schwingenden Bioreaktors (10) ist.

3. System nach Anspruch 1, wobei das Ansatzteil (13) eine Quellenhaube (31) und eine Detektorhaube (33) aufweist, die von einer Außenwand (106) des Beutels nach innen ragen, wobei eine Wand der Quellenhaube und eine Wand der Detektorhaube den Probenspalt (19) zwischen sich bilden.

4. System nach Anspruch 3, wobei die Quellenhaube (31) und die Detektorhaube (33) jeweils von außerhalb des Beutels zugänglich sind.

5. System nach Anspruch 4, wobei das Sondenmodul (15) in das Ansatzteil (13) eingeführt ist, derart, dass die Quellenhaube (31) eine Quellenbaugruppe (23) aufnimmt, in der das Quellenelement (21) untergebracht ist, und die Detektorhaube (33) eine Detektorbaugruppe (27) aufnimmt, in der das Detektorelement (25) untergebracht ist.

6. System nach Anspruch 5, wobei die Quellenbaugruppe (23) und die Detektorbaugruppe (27) jeweils an einem gemeinsamen Montageträger (45) des Sondenmoduls (15) befestigt oder mit diesem einstückig ausgeführt sind.

7. System nach Anspruch 6, wobei Größen und Formen der Quellenbaugruppe (23) und der Detektorbaugruppe (27) sowie der Abstand zwischen der Quellenbaugruppe und der Detektorbaugruppe den Größen und Formen der Quellenhaube (31) und der Detektorhaube (33) und dem Abstand dazwischen entsprechen.

8. System nach Anspruch 1, wobei das Ansatzteil (13) dafür ausgelegt ist, an der Wand (106) des Beutels mittels Schweißen, Thermoformen oder Klebstoff befestigt zu werden.

9. System nach Anspruch 1, wobei der Beutel (100, 12) und das Ansatzteil (13) Einweg- oder Einmalartikel sind und das Sondenmodul (15) mit verschiedenen Ansatzteilen und Beuteln wiederverwendbar ist.

10. System nach Anspruch 1, wobei das Ansatzteil eine oder mehrere Fensterplatten (41, 43) zum Übertragen des Lichts zwischen dem Probenspalt (19) und der Quellenhaube (31) und/oder Detektorhaube (33) aufweist.

11. Verfahren zum Überwachen eines Prozesses, der innerhalb eines Beutels (100, 12) für einen Bioreaktor durchgeführt wird, mithilfe des Systems nach Anspruch 1, wobei das Verfahren umfasst:
Befestigen des Ansatzteils (13) an einer Wand (106) des Beutels (100, 12);
Leiten von Licht vom Quellenelement (21) des Sondenmoduls (15) durch den Probenspalt (19); und
Erfassen des Lichts am Detektorelement (25) des Sondenmoduls (15) nach Wechselwirkung zwischen dem Licht und dem Inhalt (22) des Beutels.

12. Verfahren nach Anspruch 11, wobei der Beutel (100, 12) ein flexibler Beutel eines schwingenden Bioreaktors (10) ist.

13. Verfahren nach Anspruch 11, darüber hinaus umfassend, dass eine Quellenhaube (31) des Ansatzteils (13) und eine Detektorhaube (33) des Ansatzteils von einer Außenwand (106) des Beutels (100, 12) nach innen ragen, wobei eine Wand der Quellenhaube (31) und eine Wand der Detektorhaube (33) den Probenspalt (19) zwischen sich bilden.

14. Verfahren nach Anspruch 13, wobei die Quellenhaube (31) und die Detektorhaube (33) jeweils von außerhalb des Beutels zugänglich sind.

15. Verfahren nach Anspruch 14, darüber hinaus umfassend, das Sondenmodul (15) in das Ansatzteil (13) einzuführen, derart, dass die Quellenhaube (31) eine Quellenbaugruppe (23) aufnimmt, in der das Quellenelement (21) untergebracht ist, und die Detektorhaube (33) eine Detektorbaugruppe (27) aufnimmt, in der das Detektorelement (25) untergebracht ist.

16. Verfahren nach Anspruch 15, wobei die Quellenbaugruppe (23) und die Detektorbaugruppe (27) jeweils an einem gemeinsamen Montageträger (45) des Sondenmoduls befestigt oder mit diesem einstückig ausgeführt sind.

17. Verfahren nach Anspruch 16, darüber hinaus umfassend, die Größen und Formen der Quellenbaugruppe (23) und der Detektorbaugruppe (27) sowie den Abstand zwischen der Quellenbaugruppe und der Detektorbaugruppe so zu konfigurieren, dass sie den Größen und Formen der Quellenhaube (31) und der Detektorhaube (33) und dem Abstand dazwischen entsprechen.

18. Verfahren nach Anspruch 11, darüber hinaus umfassend, das Ansatzteil (13) an der Wand (106) des Beutels mittels Schweißen, Thermoformen oder Klebstoff zu befestigen.

19. Verfahren nach Anspruch 11, wobei der Beutel (100, 12) und das Ansatzteil (13) Einweg- oder Einmalartikel sind und das Sondenmodul mit verschiedenen Ansatzteilen und Beuteln wiederverwendbar ist.

20. Verfahren nach Anspruch 11, wobei das Ansatzteil eine oder mehrere Fensterplatten (41, 43) zum Übertragen des Lichts zwischen dem Probenspalt (19) und der Quellenhaube (31) und/oder Detektorhaube (33) aufweist.

21. Beutel (100, 12) für einen Bioreaktor, wobei der Beutel aufweist:
ein Ansatzteil (13), das an einer Wand (106) des Beutels befestigt ist, wobei das Ansatzteil (13) einen Probenspalt (19) bildet und mindestens eine Haube umfasst, die in den Beutel ragt, wobei ein Sondenmodul (15) zum Überwachen eines Inhalts (22) des Beutels, das ein Quellenelement (31) und ein Detektorelement (33) zum Erfassen von Licht aufweist, das nach Wechselwirkung mit dem Inhalt (22) vom Quellenelement (31) durch den Probenspalt (19) übertragen wurde, in das Ansatzteil (13) eingeführt oder mit diesem gekoppelt werden kann.

22. Schwingendes Bioreaktorsystem (10), aufweisend:
einen motorisch angetriebenen Träger (24);
einen schwingenden Bioreaktorbeutel (100, 12) nach Anspruch 21.

## Revendications

1. Système (11) destiné à surveiller un processus effectué à l'intérieur d'un sac (100, 12) pour un bioréacteur, le système comprenant :
une pièce rapportée (13) conçue pour être fixée à une paroi (106) du sac et formant un espace d'échantillonnage (19) à l'intérieur du sac, la pièce rapportée (13) comprenant au moins un capot qui fait saillie dans le sac ; et
un module de sonde (15) conçu pour être inséré dans ou couplé à la pièce rapportée (13), pour surveiller un contenu (22) du sac, le module de sonde (15) comprenant un élément de source (21) et un élément de détecteur (25) pour recevoir la lumière transmise par l'élément de source (21) à travers l'espace d'échantillonnage (19) après l'interaction avec le contenu (22).

2. Le système selon la revendication 1, sachant que le sac (100, 12) est un sac flexible d'un bioréacteur oscillant (10).

3. Le système selon la revendication 1, sachant que la pièce rapportée (13) comprend un capot de source (31) et un capot de détecteur (33) qui font saillie vers l'intérieur à partir d'une paroi extérieure (106) du sac, une paroi du capot de source et une paroi du capot de détecteur formant entre elles l'espace d'échantillonnage (19).

4. Le système selon la revendication 3, sachant que le capot de source (31) et le capot de détecteur (33) sont chacun accessibles depuis l'extérieur du sac.

5. Le système selon la revendication 4, sachant que le module de sonde (15) est inséré dans la pièce rapportée (13) de sorte que le capot de source (31) reçoit un ensemble de source (23) dans lequel est logé l'élément de source (21), et le capot de détecteur (33) reçoit un ensemble de détecteur (27) dans lequel est logé l'élément de détecteur (25).

6. Le système selon la revendication 5, sachant que l'ensemble de source (23) et l'ensemble de détecteur (27) sont chacun fixés à un support de montage commun (45) du module de sonde (15) ou sont réalisés d'un seul tenant avec celui-ci.

7. Le système selon la revendication 6, sachant que les dimensions et les formes de l'ensemble de source (23) et de l'ensemble de détecteur (27) ainsi que la distance entre l'ensemble de source et l'ensemble de détecteur correspondent aux dimensions et aux formes du capot de source (31) et du capot de détecteur (33) et à la distance entre ceux-ci.

8. Le système selon la revendication 1, sachant que la pièce rapportée (13) est conçue pour être fixée à la paroi (106) du sac par soudage, thermoformage ou collage.

9. Le système selon la revendication 1, sachant que le sac (100, 12) et la pièce rapportée (13) sont des articles jetables ou à usage unique et le module de sonde (15) est réutilisable avec différentes pièces rapportées et différents sacs.

10. Le système selon la revendication 1, sachant que la pièce rapportée comprend une ou plusieurs plaques de fenêtre (41, 43) destinées à transmettre la lumière entre l'espace d'échantillonnage (19) et le capot de source (31) et/ou le capot de détecteur (33).

11. Procédé de surveillance d'un processus effectué à l'intérieur d'un sac (100, 12) pour un bioréacteur à l'aide du système selon la revendication 1, le procédé comprenant :
la fixation de la pièce rapportée (13) à une paroi (106) du sac (100, 12) ;
le fait de diriger la lumière provenant de l'élément de source (21) du module de sonde (15) à travers l'espace d'échantillonnage (19) ; et
le fait de détecter la lumière au niveau de l'élément de détecteur (25) du module de sonde (15) après l'interaction de la lumière avec le contenu (22) du sac.

12. Le procédé selon la revendication 11, sachant que le sac (100, 12) est un sac flexible d'un bioréacteur oscillant (10).

13. Le procédé selon la revendication 11, comprenant en outre le fait qu'un capot de source (31) de la pièce rapportée (13) et un capot de détecteur (33) de la pièce rapportée font saillie vers l'intérieur à partir d'une paroi extérieure (106) du sac (100, 12), une paroi du capot de source (31) et une paroi du capot de détecteur (33) formant l'espace d'échantillonnage (19) entre elles.

14. Le procédé selon la revendication 13, sachant que le capot de source (31) et le capot de détecteur (33) sont chacun accessibles depuis l'extérieur du sac.

15. Le procédé selon la revendication 14, comprenant en outre l'insertion du module de sonde (15) dans la pièce rapportée (13) de sorte que le capot de source (31) reçoit un ensemble de source (23) dans lequel est logé l'élément de source (21), et le capot de détecteur (33) reçoit un ensemble de détecteur (27) dans lequel est logé l'élément de détecteur (25).

16. Le procédé selon la revendication 15, sachant que l'ensemble de source (23) et l'ensemble de détecteur (27) sont chacun fixés à un support de montage commun (45) du module de sonde ou sont réalisés d'un seul tenant avec celui-ci.

17. Le procédé selon la revendication 16, comprenant en outre la configuration des tailles et des formes de l'ensemble de source (23) et de l'ensemble de détecteur (27) ainsi que de la distance entre l'ensemble de source et l'ensemble de détecteur de manière à ce qu'elles correspondent aux tailles et aux formes du capot de source (31) et du capot de détecteur (33) et à la distance entre ceux-ci.

18. Le procédé selon la revendication 11, comprenant en outre la fixation de la pièce rapportée (13) à la paroi (106) du sac par soudage, thermoformage ou collage.

19. Le procédé selon la revendication 11, sachant que le sac (100, 12) et la pièce rapportée (13) sont des articles jetables ou à usage unique et le module de sonde (15) est réutilisable avec différentes pièces rapportées et différents sacs.

20. Le procédé selon la revendication 11, sachant que l'élément rapporté comporte une ou plusieurs plaques de fenêtre (41, 43) destinées à transmettre la lumière entre l'espace d'échantillonnage (19) et le capot de source (31) et/ou le capot de détecteur (33).

21. Sac (100, 12) pour un bioréacteur, le sac comprenant :
une pièce rapportée (13) fixée à une paroi (106) du sac, la pièce rapportée (13) formant un espace d'échantillonnage (19) et comprenant au moins un capot qui fait saillie dans le sac, un module de sonde (15) pour surveiller un contenu (22) du sac, qui comprend un élément de source (31) et un élément de détecteur (33) pour détecter la lumière transmise provenant de l'élément de source (31) à travers l'espace d'échantillonnage (19) après l'interaction avec le contenu (22), peut être inséré dans la pièce rapportée (13) ou couplé à celle-ci.

22. Système de bioréacteur oscillant (10) comprenant :
un support entraîné par un moteur (24) ;
un sac de bioréacteur oscillant (100, 12) selon la revendication 21.
